# FASCICULE DE BREVET EUROPEEN

(11) **EP 1 025 839 B1**
(45) Date de publication et mention de la délivrance du brevet: **21.07.2004**
(21) Numéro de dépôt: 00400052.7
(22) Date de dépôt: 11.01.2000
(51) Int. Cl.: A61K 7/50, A61K 7/06, A61K 7/02

(54) **Composition cosmétique comprenant un tensioactif anionique, un tensioactif amphotère, une polyoléfine, un polymère cationique et un sel ou un alcool hydrosoluble, utilisation et procédé**
Kosmetische Zusammensetzung enthaltend ein anionisches und amphoteres Tensid, ein Polyolefin, ein kationisches Polymer und ein Salz oder einen wasserlöslichen Alkohol, Verwendung und Verfahren
Cosmetic composition containing an antionic and amphoteric surfactant, a polyolefin, a cationic polymer and a salt or watersoluble alcohol, use and process.

(30) Priorité: 03.02.1999 FR 9901239
(43) Date de publication de la demande: 09.08.2000
(73) Titulaire: L'OREAL, 75008 Paris (FR)
(72) Inventeur: Restle, Serge, 95390 Saint Prix (FR); Garnier, Nathalie, Springfield, NJ 07081 (US)
(74) Mandataire: Le Blainvaux Bellegarde, Françoise

(56) Documents cités:
- EP-A- 0 413 417
- EP-A- 0 422 862
- WO-A-97/35542
- WO-A-97/35549

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, au moins un tensioactif anionique, au moins un tensioactif amphotère et au moins une huile de type polyoléfine, au moins un polymère cationique et au moins un sel ou un alcool hydrosoluble, le rapport en poids tensioactif anionique / tensioactif amphotère étant inférieur ou égal à 3. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétée, les fibres capillaires. Ces agents conditionneurs peuvent bien entendu également améliorer le comportement cosmétique des cheveux naturels.

Dans ce but, on a déjà proposé d'utiliser des agents conditionneurs insolubles. Ces composés insolubles présentent l'inconvénient d'être difficiles à maintenir en dispersion régulière dans le milieu.

Pour les maintenir en suspension, on a déjà proposé l'utilisation des dérivés d'esters ou d'éthers à longue chaîne (agents nacrants) ou des polysaccharides tels que la gomme de xanthane (gélifiants). Cependant, les agents nacrants présentent des problèmes de cristallisation qui entraînent parfois une évolution (augmentation) de la viscosité des compositions au cours du temps ; les agents gélifiants présentent également des inconvénients, à savoir d'une part que la mousse des compositions détergentes contenant des polysaccharides se développe difficilement (mauvais démarrage de mousse) et que, d'autre part, les compositions n'ont pas une texture lisse et s'écoulent par paquets, ce qui est peu apprécié des utilisateurs. De plus, ces divers agents de suspension ne permettent pas d'obtenir des compositions transparentes ou limpides.

La présente invention a pour but de proposer des compositions ne présentant pas les inconvénients des compositions citées ci-dessus.

Les agents conditionneurs de type polyoléfine doivent également être véhiculées sur les matières kératiniques traitées en vue de leur conférer, suivant l'application, des propriétés de douceur, de brillance et de démêlage sans induire de caractère gras.

Ainsi, à la suite d'importantes recherches menées sur la question, il a maintenant été trouvé par la Demanderesse, qu'en utilisant une base lavante particulière, au moins une huile de type polyoléfine particulière, au moins un polymère cationique et au moins un sel hydrosoluble ou un alcool mono ou polyvalent hydrosoluble, il est possible d'obtenir des compositions détergentes stables et transparentes présentant d'excellentes propriétés cosmétiques, en particulier le démêlage et le lissage des cheveux traités et ayant de bonnes propriétés d'usage tel qu'un bon pouvoir lavant intrinsèque et un bon pouvoir moussant.

La mise en oeuvre industrielle est extrêmement facile et les propriétés cosmétiques des shampooings sont excellentes.

Les compositions obtenues sont stables au stockage, sans nécessiter l'addition d'agent de dispersion et/ou de mise en suspension de l'huile selon l'invention.

En l'absence de composés additionnels insolubles, les compositions obtenues sont également transparentes. Elles peuvent contenir des quantités importantes d'huile de type polyoléfine tout en conservant une bonne transparence et en ayant de bonnes propriétés cosmétiques.

Les compositions conformes à l'invention confèrent aux cheveux, après rinçage, un remarquable effet traitant qui se manifeste notamment par une facilité de démêlage, ainsi qu'un apport de lissage, de douceur et de souplesse sans aucune sensation de gras.

Ainsi, la présente invention a pour objet de nouvelles compositions cosmétiques détergentes et conditionnantes caractérisée par le fait qu'elles comprennent, dans un milieu aqueux cosmétiquement acceptable, (A) une base lavante comprenant, au moins un tensioactif anionique, au moins un tensioactif amphotère, (B) au moins une huile de type polyoléfine de poids moléculaire inférieur à 450, (C) au moins un polymère cationique, (D) au moins un sel hydrosoluble et/ou un alcool hydrosoluble mono ou polyhydroxylé, le rapport en poids tensioactif anionique / tensioactif amphotère étant inférieur ou égal à 3.

L'invention a également pour objet l'utilisation en cosmétique des compositions ci-dessus pour le nettoyage et /ou le démaquillage et/ou le conditionnement des matières kératiniques telles que les cheveux et la peau.

### A- BASE LAVANTE :

La base lavante comprend un ou plusieurs tensioactifs anioniques et un ou plusieurs tensioactifs amphotères.

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium.

Parmi les tensioactifs anioniques, on préfère utiliser selon l'invention les sels d'alkylsulfates et d'alkyléthersulfates et leurs mélanges.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL®, tels que décrits dans les brevets US-2 528 378 et US-2 781 354 et de structures:

R₂-CONHCH₂CH₂-N(R₃)(R₄)(CH₂COO-) (I)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R_{2'}-CONHCH₂CH₂-N(B)(C) (II)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂ - CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHONE POULENC.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines telles que la TEGOBETAINE® F50 commercialisée par la société GLODSCHMIDT.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

A titre indicatif, les compositions détergentes conformes à l'invention présentent généralement les compositions suivantes:
(i) tensio-actif(s) anionique(s) : de 1 à 30 % en poids, de préférence de 5 à 20 % en poids et plus particulièrement de 5 à 12% en poids, par rapport au poids total de la composition détergente;
(ii) tensio-actif(s) amphotère(s): de 0,15 à 20 % en poids, de préférence de 1,5 à 15 % en poids et plus particulièrement de 3 à 15% en poids, par rapport au poids total de la composition.

Le rapport en poids tensioactif anionique / tensioactif amphotère est de préférence compris entre 0,2 et 3 plus particulièrement entre 0,4 et 2,5.

### B- Polyoléfines

Les compositions selon l'invention comprennent nécessairement une huile de type polyoléfine de poids moléculaire inférieur à 450, de préférence compris entre 150 et 350.

Les polyoléfines sont de préférence des poly-α-oléfines et en particulier:
- de type polybutène, hydrogéné ou non, et de préférence polyisobutène, hydrogéné ou non.
   On utilise de préférence les oligomères d'isobutylène de poids moléculaire inférieur à 450.
   A titre d'exemples de poly-α-oléfines utilisables dans le cadre de la présente invention, on peut plus particulièrement mentionner les produits vendus sous le nom de PERMETHYL 99 A, 101 A, 102 A par la Société PRESPERSE Inc, ou bien encore les produits vendus sous le nom de ARLAMOL HD par la Société ICI,
- de type polydécène, hydrogéné ou non.
   De tels produits sont vendus par exemple sous les dénominations ETHYLFLO par la société ETHYL CORP., et d'ARLAMOL PAO par la société ICI.

La ou les huiles de type polyoléfine peuvent être utilisées dans les compositions conformes à l'invention dans des concentrations généralement comprises entre 0,5 et 15 %, et de préférence entre 1 et 10 % en poids par rapport au poids total de la composition et encore plus particulièrement de 2 à 8% en poids.

### C) Polymères cationiques

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Les polymères cationiques utilisables selon l'invention ont généralement une densité de charge cationique supérieure à 0,2 meq./g.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer:
(1) Les copolymères vinylpyrrolidone-acrylate ou -méthacrylate de dialkylamino-alkyle quaternisés ou non, tels que les produits vendus sous la dénomination "Gafquat" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclopolymères alkyl diallyl amine ou de dialkyl diallyl ammonium tels que les homopolymères ou copolymères comportant comme constituant principal de la chaîne des motifs répondant aux formules (III) ou (IV): 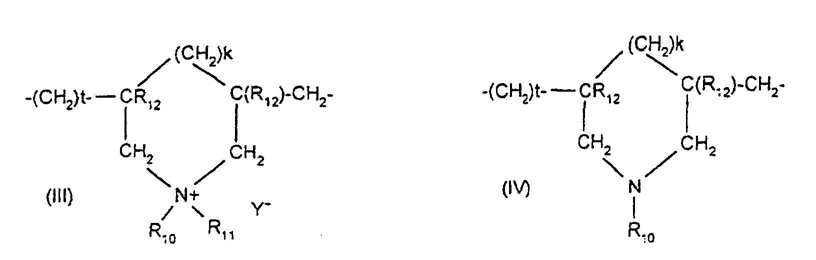 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Merck et ses homologues de faibles masses moléculaires moyenne en poids.
(10) le polymère de diammonium quaternaire contenant des motifs récurrents répondant à la formule: 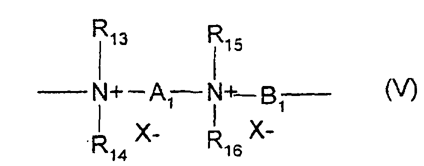 formule (V) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne:
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine;
   c) un reste de diamine bis-primaire de formule: -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH- ;

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   Selon l'invention, on peut utiliser plus particulièrement les polymères choisis parmi le composés de formule (V) dans laquelle R₁₃, R₁₄, R₁₅ et R₁₆ représentent le radical méthyle, A1 représente le radical de formule -(CH₂)₃- et B1 représente le radical de formule -(CH2)6- et X⁻ représente l'anion chlorure et le composé de formule (V) dans laquelle R₁₃ et R₁₄ représentent le radical éthyle, R₁₅ et R₁6 représentent le radical méthyle, A1 et B1 représentent le radical de formule -(CH₂)₃- et X⁻ représente l'anion bromure.
(11) les polymères de polyammonium quaternaires constitués de motifs de formule (VI): 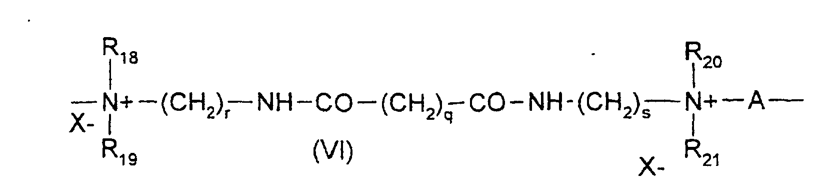 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol A 15", "Mirapol AD1", "Mirapol AZ1" et "Mirapol 175" vendus par la société Miranol.
(12) les homopolymères ou copolymères dérivés des acides acrylique ou méthacrylique et comportant des motifs choisis parmi : 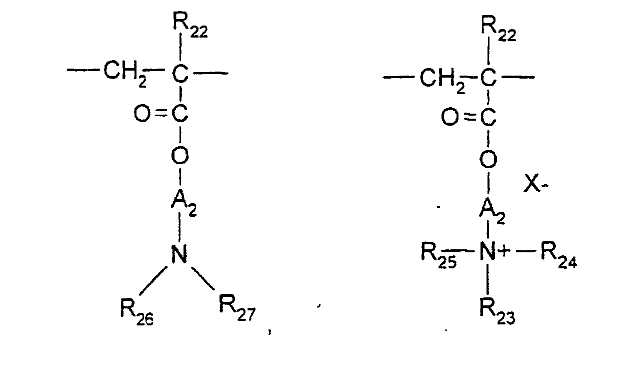 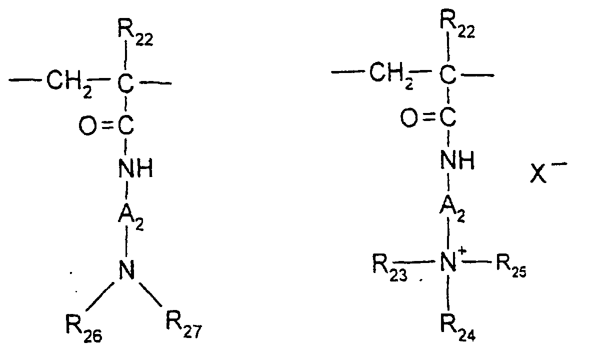 dans lesquels les groupements R₂₂ désignent indépendamment H ou CH₃,
   les groupements A2 désignent indépendamment un groupe alkyle linéaire ou ramifié de 1 à 6 atomes de carbone ou un groupe hydroxyalkyle de 1 à 4 atomes de carbone,
   les groupements R₂₃, R₂₄, R₂₅, identiques ou différents, désignant indépendamment un groupe alkyle de 1 à 18 atomes de carbone ou un radical benzyle,
   les groupements R₂₆ et R₂₇ représentent un atome d'hydrogène ou un groupement alkyle de 1 à 6 atomes de carbone,
   X⁻ désigne un anion, par exemple méthosulfate ou halogénure, tel que chlorure ou bromure.
   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations Luviquat FC 905, FC 550 et FC 370 par la société B.A.S.F..
(14) Les polyamines comme le Polyquart H vendu par HENKEL, référencé sous le nom de « POLYETHYLENEGLYCOL (15) TALLOW POLYAMIDE » dans le dictionnaire CTFA.

(12) Les polymères de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant éventuellement suivie d'une réticulation par un composé à insaturation oléfinique, en particulier le méthylène bis acrylamide, le diméthacrylate d'éthylèneglycol ou le triméthacrylate de pentaérythritol. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 et SC96» par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères de vinylpyrrolidone et de sel de vinyl imidazoline tels que les produits commercialisés par BASF sous les dénominations LUVIQUAT FC 370, LUVIQUAT FC 550, LUVIQUAT FC 905 et LUVIQUAT HM-552.

On peut également utiliser les polymères qui sont constitués de motifs récurrents répondant à la formule: 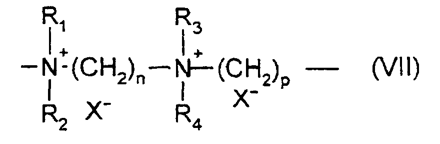 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (VII) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).

Selon l'invention, le ou les polymères cationiques peuvent représenter de 0,05 % à 10 % en poids, de préférence de 0,1 % à 5 % en poids, et encore plus préférentiellement de 0,25 % à 3 % en poids, du poids total de la composition finale.

### D) Sels et alcools hydrosolubles

Les sels hydrosolubles selon l'invention sont de préférence les sels de métaux mono ou divalents et d'un acide minéral ou organique.
On peut citer en particulier le chlorure de sodium, le chlorure de potassium, le chlorure de calcium, le sulfate de magnésium, le citrate de sodium, les sels de sodium de l'acide phosphorique. De préférence, on utilise les sels de métaux monovalents. Le chlorure de sodium est particulièrement préféré

Les sels hydrosolubles sont présents généralement à des concentrations comprises entre 0,1 et 10% en poids et de préférence entre 0,5 et 5% en poids par rapport au poids total de la composition.

Les alcools hydrosolubles mono ou polyhydroxylés sont notamment les alcools inférieurs en C₁-C₆, comme l'éthanol, l'isopropanol, le tertiobutanol, le n-butanol, les polyols tels que les alkylèneglycols comme le propylèneglycol, la glycérine et les polyalkylèneglycols ; les éthers de glycols.

Le ou les alcools hydrosolubles peuvent être utilisés dans des concentrations généralement comprises entre 0,1 et 20% en poids et plus particulièrement entre 0,2 et 10% en poids par rapport au poids total de la composition.

Les compositions détergentes selon l'invention présentent un pH final généralement compris entre 3 et 8. De préférence, ce pH est compris entre 4 et 6,5. L'ajustement du pH à la valeur désirée peut se-faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence l'acide citrique ou l'acide chlorhydrique.

Le milieu aqueux cosmétiquement acceptable peut être constitué uniquement par de l'eau ou par un mélange d'eau et d'un solvant cosmétiquement acceptable.

Les compositions conformes à l'invention peuvent contenir en plus de l'association définie ci-dessus des agents régulateurs de viscosité tels que des électrolytes, ou des agents épaississants. On peut citer en particulier le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcools gras, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques des cheveux ou de la peau sans cependant altérer la stabilité des compositions. On peut citer à ce sujet les agents tensioactifs cationiques, les polymères anioniques ou non ioniques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoïque, les hydroxyacides, les vitamines, le panthénol, les silicones volatiles ou non volatiles, solubles ou insolubles dans le milieu, les filtres UV, les agents hydratants, les agents antipelliculaires ou antiséborrhéiques, les agents anti-radicaux libres, et leurs mélanges.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle la stabilité des compositions et les propriétés cosmétiques attachées intrinsèquement aux compositions conformes à l'invention ne soient pas, ou substantiellement pas, altérées par la ou les adjonctions envisagées.

La transparence peut se mesurer par la turbidité au turbidimètre HACH - Modèle 2100 P à 25°C (L'appareil est étalonné avec de la formazine). La turbidité des compositions selon l'invention (en l'absence de composés additionnels insolubles) est alors généralement comprise entre 0,05 et 50 NTU et de préférence inférieure à 25 NTU. Lorsque l'huile est sous forme de globules, la taille de ces globules d'huile est de préférence inférieure à 5 nanomètres.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes:
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage à l'eau, l'opération pouvant être répétée une ou plusieurs fois.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés.

### EXEMPLE 1

On a réalisé deux compositions de shampooings, l'une conforme à l'invention (composition A) et l'autre comparative (composition B) :

| | B Comparatif | A Invention |
|---|---|---|
| - Lauryléthersulfate de sodium (C12/C14 à 70/30) à 2,2 moles d'oxyde d'éthylène en solution aqueuse à 70% de MA | 17,5 gMA | 7,5 gMA |
| - Cocoylbétaïne (DEHYTON AB 30) | 2,5 gMA | 12,5 gMA |
| - 2,2,4,4,6,6,8-heptaméthylnonane (Isohexadécane de BAYER) | 2 g | 2 g |
| - Homopolymère de chlorure de diallyl diméthyl ammonium en solution aqueuse à 40% de MA (MERQUAT 100 de CALGON) | 0,4 gMA | 0,4 gMA |
| - NaCl | 4 g | 4 g |
| - Parfum, conservateur | qs | qs |
| - Acide chlorhydrique qs pH | 6 | 6 |
| - Eau déminéralisée qs | 100 g | 100 g |

La composition A selon l'invention (TA anionique /TA amphotère = 0,6) est transparente. Turbidité = 31,4 NTU
La composition B (TA anionique /TA amphotère = 7) n'est pas transparente : Turbidité = >900 NTU
(La transparence est évaluée par turbidimétrie en unités NTU (Nephelometric turbidity units).)

On a effectué un shampooing en appliquant environ 1 g de la composition A sur des mèches de cheveux sensibilisés (2,5 g) préalablement mouillés. On fait mousser le shampooing, on laisse poser 10 mn puis on rince abondamment à l'eau. On essore les mèches.
On procède selon le même mode opératoire que ci-dessus avec la composition comparative B.

Un panel d'experts a évalué l'aspect des cheveux séchés 10 minutes à 60°C.

Tous les experts indiquent que les cheveux traités avec la composition A selon l'invention se démêlent plus facilement, sont plus doux et plus lisses que les cheveux traités avec la composition B.

### EXEMPLE 2

On a réalisé deux compositions de shampooings, l'une conforme à l'invention (composition A) et l'autre comparative (composition B):

| | B Comparatif | A Invention |
|---|---|---|
| -Acide lauryl éther carboxylique polyoxyéthyléné en solution aqueuse à 90% de MA (AKYPO RLM 45 CA de KAO | 8 gMA | 8 gMA |
| - Cocoamphocarboxyglycinate de sodium en solution aqueuse à 38% de MA (MIRANOL C2M Conc. de RHODIA CHIMIE) | 4 gMA | 4 gMA |
| - 2,2,4,4,6,6,8-heptaméthylnonane (Isohexadécane de BAYER) | 1 g | 1 g |
| - Homopolymère de chlorure de diallyl diméthyl ammonium en solution aqueuse à 40% de MA (MERQUAT 100 de CALGON) | - | 0,4 gMA |
| - NaCl | - | 4 g |
| - Parfum, conservateur | qs | qs |
| - Acide chlorhydrique qs pH | 6 | 6 |
| - Eau déminéralisée qs | 100 g | 100 g |

On a effectué un shampooing en appliquant environ 1 g de la composition A sur des mèches de cheveux sensibilisés (2,5 g) préalablement mouillés. On fait mousser le shampooing, on laisse poser 10 mn puis on rince abondamment à l'eau. On essore les mèches.
On procède selon le même mode opératoire que ci-dessus avec la composition comparative B.

Un panel d'experts a évalué l'aspect des cheveux séchés 10 minutes à 60°C. Tous les experts indiquent que les cheveux traités avec la composition A selon l'invention se démêlent plus facilement, sont plus doux et plus lisses que les cheveux traités avec la composition B.

### EXEMPLE 3

On a réalisé deux compositions de shampooings, l'une conforme à l'invention (composition A) et l'autre comparative (composition B):

| | B(Comparatif) | A(Invention) |
|---|---|---|
| - Lauryléthersulfate d'ammonium à 3 moles d'oxyde d'éthylène en solution aqueuse à 70% de MA (MA = matière active) | 10 gMA | 8,33 gMA |
| - Cocoamidopropylbétaïne | 3 gMA | 5 gMA |
| - Laurylsulfosuccinate de sodium | 2 gMA | 1,67 gMA |
| - Iso-eicosane (PERMETHYL 102 A) | 0,5 g | 0,5 g |
| - Copolymère de chlorure de diallyl diméthyl ammonium et d'acrylamide en solution aqueuse à 8% de MA (MERQUAT 550 de CALGON) | 0,2 gMA | 0,2 gMA |
| - NaCl | - | 2 g |
| - Parfum, conservateur | qs | qs |
| - Acide chlorhydrique qs pH | 6 | 6 |
| - Eau déminéralisée qs | 100 g | 100 g |

La composition A selon l'invention (TA anionique /TA amphotère = 2) est transparente. Turbidité = 6,06 NTU
La composition B (TA anionique /TA amphotère =4) n'est pas transparente : Turbidité = 110 NTU

On a effectué un shampooing en appliquant environ 1 g de la composition A sur des mèches de cheveux sensibilisés (2,5 g) préalablement mouillés. On fait mousser le shampooing, on laisse poser 10 mn puis on rince abondamment à l'eau. On essore les mèches.
On procède selon le même mode opératoire que ci-dessus avec la composition comparative B.

Un panel d'experts a évalué l'aspect des cheveux séchés 10 minutes à 60°C.
90% des experts indiquent que les cheveux traités avec la composition A selon l'invention sont plus doux et plus lisses que les cheveux traités avec la composition B.

## Revendications

1. Composition cosmétique détergente et conditionnante, **caractérisée par le fait qu'**elle comprend, dans un milieu aqueux cosmétiquement acceptable, (A) une base lavante comprenant, au moins un tensioactif anionique, au moins un tensioactif amphotère, (B) au moins une huile de type polyoléfine de poids moléculaire inférieur à 450, (C) au moins un polymère cationique, (D) au moins un sel hydrosoluble et/ou un alcool hydrosoluble mono ou polyhydroxylé, le rapport en poids tensioactif anionique / tensioactif amphotère étant inférieur ou égal à 3.

2. Composition selon la revendication 1, **caractérisée par le fait que** ladite base lavante est présente à une teneur pondérale comprise entre 4 % et 50 % en poids par rapport au poids total de la composition, de préférence comprise entre 6 % et 35 % en poids et plus préférentiellement comprise entre 8 % et 25 % en poids.

3. Composition selon l'une quelconque des revendications 1 ou 2, **caractérisée en ce que** le(s) tensioactif(s) anionique(s) est (sont) présent(s) dans des concentrations allant de 1 à 30 % en poids, de préférence de 5 à 20 % en poids, par rapport au poids total de la composition.

4. Composition selon l'une quelconque des revendications 1 à 3, **caractérisée en ce que** le(s) tensioactif(s) amphotère(s) est(sont) présent(s) dans des concentrations allant de 0,15 à 20 % en poids, de préférence de 1,5 à 15 % en poids, par rapport au poids total de la composition.

5. Composition selon l'une quelconque des revendications 1 à 4, caractérisée le rapport en poids tensioactif anionique / tensioactif amphotère est compris entre 0,2 et 3, plus particulièrement entre 0,4 et 2,5.

6. Composition selon l'une quelconque des revendications 1 à 5, **caractérisée en ce que** ladite huile de type polyoléfine présente un poids moléculaire compris entre 150 et 350.

7. Composition selon l'une quelconque des revendications 1 à 6, **caractérisée en ce que** ladite huile de type polyoléfine est une poly-α-oléfine.

8. Composition selon la revendication 7, **caractérisée en ce que** ladite huile de type poly-α-oléfine est choisie parmi les huiles de type:
- de type polybutène, hydrogéné ou non, et de préférence polyisobutène, hydrogéné ou non.
- de type polydécène, hydrogéné ou non.

9. Composition selon l'une quelconque des revendications 1 à 8, **caractérisée par le fait que** l'huile de type polyoléfine est présente dans des concentrations comprises entre 0,5 et 15 %, et de préférence entre 1 et 10 % en poids par rapport au poids total de la composition.

10. Composition selon l'une quelconque des revendications 1 à 9, **caractérisée par** le fait ledit polymère cationique est choisi parmi :
(1) Les copolymères vinylpyrrolidone-acrylate ou -méthacrylate de dialkylamino-alkyle quaternisés ou non,
(2) Les dérivés d'éthers de cellulose comportant des groupements ammonium quaternaires
(3) Les dérivés de cellulose cationiques tels que les copolymères de cellulose ou les dérivés de cellulose greffés avec un monomère hydrosoluble d'ammonium quaternaire,
(4) Les polysaccharides tels que les gommes de guar contenant des groupements cationiques trialkylammonium.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères.
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées.
(7) les dérivés de polyaminoamides résultant de la condensation de polyalcoylènes polyamines avec des acides polycarboxyliques suivie d'une alcoylation par des agents bifonctionnels.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1.
(9) les cyclopolymères de méthyl diallyl amine ou de diméthyl diallyl ammonium tels que les homopolymères ou copolymères comportant comme constituant principal de la chaîne des motifs répondant aux formules (III) ou (IV) : 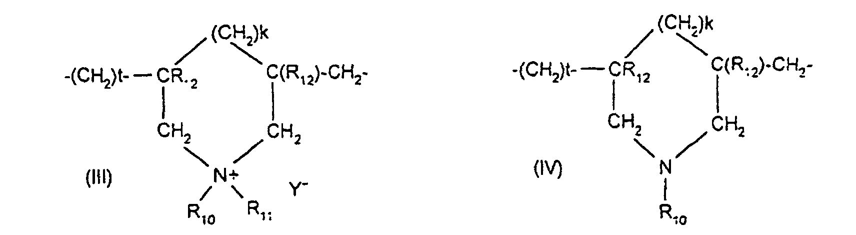 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.
(10) le polymère de diammonium quaternaire contenant des motifs récurrents répondant à la formule: 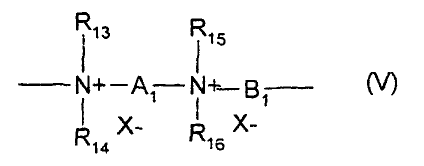 formule (V) dans laquelle :
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)ₙ-CO-D-OC-(CH₂)ₙ-
dans lequel D désigne :
a) un reste de glycol de formule : -O-Z-O-, où Z désigne un radical hydrocarboné linéaire ou ramifié ou un groupement répondant à l'une des formules suivantes :
-(CH₂CH₂O)ₓ-CH₂CH₂-
-[CH₂-CH(CH₃)-O]y-CH₂-CH(CH₃)-
où x et y désignent un nombre entier de 1 à 4, représentant un degré de polymérisation défini et unique ou un nombre quelconque de 1 à 4 représentant un degré de polymérisation moyen;
b) un reste de diamine bis-secondaire tel qu'un dérivé de pipérazine;
c) un reste de diamine bis-primaire de formule: -NH-Y-NH-, où Y désigne un radical hydrocarboné linéaire ou ramifié, ou bien le radical bivalent
-CH₂-CH₂-S-S-CH₂-CH₂- ;
d) un groupement uréylène de formule : -NH-CO-NH- ;
(11) les polymères de polyammonium quaternaires constitués de motifs de formule (VI): 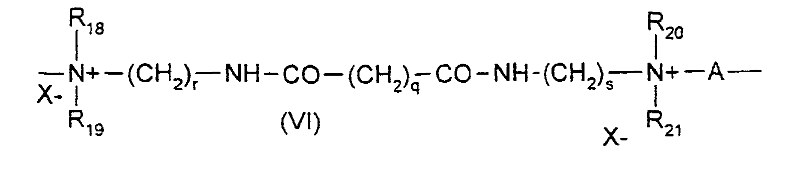 formule dans laquelle:
R₁₈, R₁₉, R₂₀ et R₂₁, identiques ou différents, représentent un atome d'hydrogène ou un radical méthyle, éthyle, propyle. β-hydroxyéthyle, β-hydroxypropyle ou -CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH₂-CH₂-O-CH₂-CH₂-.
(12) les homopolymères ou copolymères dérivés des acides acrylique ou méthacrylique et comportant des motifs choisis parmi : 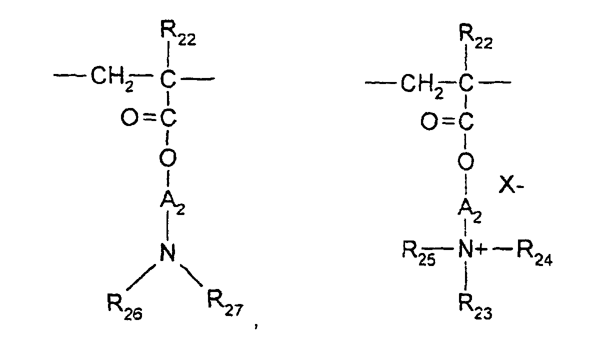 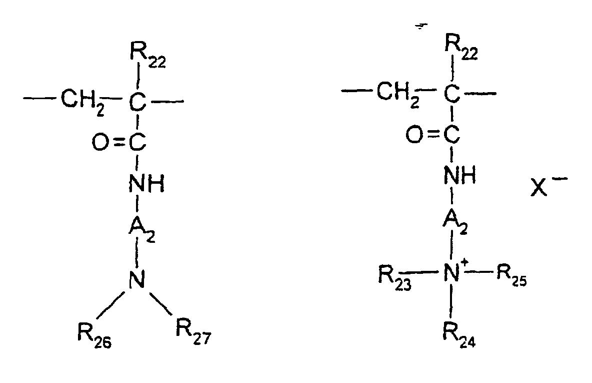 dans lesquels les groupements R₂₂ désignent indépendamment H ou CH₃,
les groupements A2 désignent indépendamment un groupe alkyle linéaire ou ramifié de 1 à 6 atomes de carbone ou un groupe hydroxyalkyle de 1 à 4 atomes de carbone,
les groupements R₂₃, R₂₄, R₂₅, identiques ou différents, désignant indépendamment un groupe alkyle de 1 à 18 atomes de carbone ou un radical benzyle.
les groupements R₂₆ et R₂₇ représentent un atome d'hydrogène ou un groupement alkyle de 1 à 6 atomes de carbone,
X⁻ désigne un anion, par exemple méthosulfate ou halogénure, tel que chlorure ou bromure.
(13) Les polymères quaternaires de vinylpyrrolidone et de vinylimidazole,
(14) Les polyamines,
(15) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.
16) des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

11. Composition selon l'une des revendications 10, **caractérisée par** le fait ledit polymère cationique est choisi parmi :
- les cyclopolymères, en particulier les homopolymères de sel de diallyldiméthylammonium et les copolymères de sel de diallyldiméthylammonium et d'acrylamide,
- les homopolymères et les copolymères éventuellement réticulés de sel de (méth)acryloyloxyéthyltriméthylammonium,
- les copolymères de vinylpyrrolidone et de sel de vinyl imidazole,
- les polymères qui sont constitués de motifs récurrents répondant à la formule: 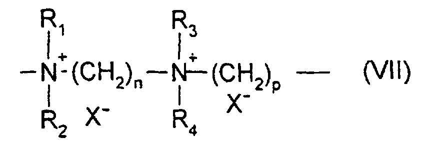
dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

12. Composition selon l'une quelconque des revendications 1 à 11, **caractérisée en ce que** ledit polymère cationique représente de 0,05 % à 10 % en poids, de préférence de 0,1 % à 5 % en poids, et encore plus préférentiellement de 0,25 % à 3 % en poids, du poids total de la composition.

13. Composition selon l'une quelconque des revendications 1 à 12, **caractérisée par** le fait les sels hydrosolubles sont des sels de métaux mono ou divalents et d'un acide minéral ou organique

14. Composition selon la revendication 13, **caractérisée par** le fait les sels hydrosolubles sont choisis parmi le chlorure de sodium, le chlorure de potassium, le chlorure de calcium, le sulfate de magnésium, le citrate de sodium, les sels de sodium de l'acide phosphorique.

15. Composition selon l'une quelconque des revendications 1 à 14, **caractérisée par le fait que** les sels hydrosolubles sont présents à des concentrations comprises entre 0,1 et 10% en poids et de préférence entre 0,5 et 5% en poids par rapport au poids total de la composition.

16. Composition selon l'une quelconque des revendications 1 à 15, **caractérisée par le fait que** les alcools hydrosolubles mono ou polyhydroxylés sont les alcools inférieurs en C₁-C₆, comme l'éthanol, l'isopropanol, le tertiobutanol, le n-butanol, les polyols tels que les alkylèneglycols comme le propylèneglycol, la glycérine et les polyalkylèneglycols ; les éthers de glycols.

17. Composition selon l'une quelconque des revendications 1 à 16, **caractérisée en ce que** les alcools hydrosolubles sont utilisés dans des concentrations généralement comprises entre 0,1 et 20% en poids et plus particulièrement entre 0,2 et 10% en poids par rapport au poids total de la composition.

18. Composition selon l'une quelconque des revendications 1 à 17, **caractérisée par le fait qu'**elle contient en plus un ou plusieurs adjuvants choisis par les agents tensioactifs cationiques, les polymères anioniques ou non ioniques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoïque, les hydroxyacides, les vitamines, le panthénol, les silicones volatiles ou non volatiles, solubles ou insolubles dans le milieu, les filtres UV, les agents hydratants, les agents antipelliculaires ou antiséborrhéiques, les agents anti-radicaux libres, et leurs mélanges.

19. Utilisation d'une composition telle définie dans l'une quelconque des revendications 1 à 18 pour le nettoyage et/ou le démaquillage et/ou le conditionnement des matières kératiniques.

20. Procédé de lavage et de conditionnement des matières kératiniques telles que les cheveux consistant à appliquer sur lesdites matières mouillées une quantité efficace d'une composition telle que définie dans l'une quelconque des revendications 1 à 18, puis à effectuer un rinçage à l'eau après un éventuel temps de pause.

## Patentansprüche

1. Reinigende und konditionierende kosmetische Zusammensetzung, **dadurch gekennzeichnet, dass** sie in einem kosmetisch akzeptablen Medium (A) eine reinigende Basisformulierung, die mindestens einen anionischen grenzflächenaktiven Stoff und mindestens einem amphoteren grenzflächenaktiven Stoff enthält, (B) mindestens ein Öl vom Polyolefintyp mit einer Molmasse unter 450, (C) mindestens ein kationisches Polymer und (D) mindestens ein wasserlösliches Salz und/oder mindestens einen ein- oder mehrwertigen, wasserlöslichen Alkohol enthält, wobei das Verhältnis anionischer grenzflächenaktiver Stoff/amphoterer grenzflächenaktiver Stoff höchstens 3 beträgt.

2. Zusammensetzung nach Anspruch 1, **dadurch gekennzeichnet, dass** die reinigende Basisformulierung in einem Gewichtsanteil von 4 bis 50 Gew.-%, bezogen auf das Gesamtgewicht der Zusammensetzung, vorzugsweise 6 bis 35 Gew.-% und besonders bevorzugt 8 bis 25 Gew.-% enthalten ist.

3. Zusammensetzung nach einem der Ansprüche 1 oder 2, **dadurch gekennzeichnet, dass** der oder die anionische(n) grenzflächenaktive(n) Stoff(e) in Konzentrationen von 1 bis 30 Gew.-% und vorzugsweise 5 bis 20 Gew.-%, bezogen auf das Gesamtgewicht der Zusammensetzung, enthalten sind.

4. Zusammensetzung nach einem der Ansprüche 1 bis 3, **dadurch gekennzeichnet, dass** der oder die amphotere(n) grenzflächenaktive(n) Stoff(e) in Konzentrationen von 0,15 bis 20 Gew.-% und vorzugsweise 1,5 bis 15 Gew.-%, bezogen auf das Gesamtgewicht der Zusammensetzung, enthalten sind.

5. Zusammensetzung nach einem der Ansprüche 1 bis 4, **dadurch gekennzeichnet, dass** das Gewichtsverhältnis anionischer grenzflächenaktiver Stoff/amphoterer grenzflächenaktiver Stoff im Bereich von 0,2 bis 3 und insbesondere 0,4 bis 2,5 liegt.

6. Zusammensetzung nach einem der Ansprüche 1 bis 5, **dadurch gekennzeichnet, dass** das Öl vom Polyolefintyp eine Molmasse von 150 bis 350 besitzt.

7. Zusammensetzung nach einem der Ansprüche 1 bis 6, **dadurch gekennzeichnet, dass** das Öl vom Polyolefintyp ein Poly-α-olefin ist.

8. Zusammensetzung nach Anspruch 7, **dadurch gekennzeichnet, dass** das Öl vom Poly-α-olefintyp unter den Ölen des folgenden Typs ausgewählt ist:
- vom Typ Polybuten, hydriert oder nicht hydriert, vorzugsweise vom Typ Polyisobuten, hydriert oder nicht hydriert,
- vom Typ Polydecen, hydriert oder nicht hydriert.

9. Zusammensetzung nach einem der Ansprüche 1 bis 8, **dadurch gekennzeichnet, dass** das Öl vom Polyolefintyp in Konzentrationen von 0,5 bis 15 Gew.-% und vorzugsweise 1 bis 10 Gew.-%, bezogen auf das Gesamtgewicht der Zusammensetzung, enthalten sind.

10. Zusammensetzung nach einem der Ansprüche 1 bis 9, **dadurch gekennzeichnet, dass** das kationische Polymer ausgewählt ist unter:
(1) Copolymeren von Vinylpyrrolidon und Dialkylaminoalkylacrylat oder Dialkylaminoalkylmethacrylat, die gegebenenfalls quaternisiert sind;
(2) Derivaten von Celluloseethern, die quartäre Ammoniumgruppen enthalten;
(3) Kationischen Cellulosederivaten, wie Cellulosecopolymeren oder Cellulosederivaten, die mit einem wasserlöslichen quartären Ammoniummonomer gepfropft sind;
(4) Polysacchariden, beispielsweise Guargummen mit kationischen Trialkylammoniumgruppen;
(5) Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder aromatische oder heterocyclische Ringe unterbrochen sind, sowie den Oxidationsprodukten und/ oder Quaternisierungsprodukten dieser Polymere;
(6) Wasserlöslichen Polyaminoamiden, die insbesondere durch Polykondensation einer sauren Verbindung mit einem Polyamin hergestellt sind; diese Polyaminoamide können mit einem Epihalohydrin, Diepoxid, Dianhydrid, ungesättigten Dianhydrid, zweifach ungesättigten Derivat, Bis-halohydrin, Bis-azetidinium, Bis-haloacyldiamin, Alkyl-bis-halogenid oder auch einem Oligomer vernetzt sein, das bei der Umsetzung einer bifunktionell reagierenden Verbindung entsteht, die gegenüber einem Bis-halohydrin, Bis-azetidinium, Bis-haloacyldiamin, Alkyl-bis-halogenid, Epihalohydrin, Diepoxid oder zweifach ungesättigten Derivat reaktiv ist, wobei das Vernetzungsmittel in Mengenanteilen von 0,025 bis 0,35 mol pro Aminogruppe des Polyaminoamids eingesetzt wird; diese Polyaminoamide können alkyliert oder, wenn sie eine oder mehrere tertiäre Aminogruppen enthalten, quaternisiert sein;
(7) Derivaten von Polyaminoamiden, die bei der Kondensation von Polyalkylenpolyaminen mit Polycarbonsäuren und anschließender Alkylierung mit bifunktionellen Mitteln entstehen;
(8) Polymeren, die durch Umsetzung eines Polyalkylenpolyamins, das zwei primäre Aminogruppen und mindestens eine sekundäre Aminogruppe aufweist, mit einer Dicarbonsäure hergestellt sind, welche unter Diglykolsäure und den gesättigten, aliphatischen Dicarbonsäuren mit 3 bis 8 Kohlenstoffatomen ausgewählt ist, wobei das Molverhältnis von Polyalkylenpolyamin und Dicarbonsäure im Bereich von 0,8 : 1 bis 1,4 : 1 liegt und das resultierende Polyaminoamid mit Epichlorhydrin in einem Molverhältnis von Epichlorhydrin zu den sekundären Aminogruppen des Polyaminoamids im Bereich von 0,5 : 1 bis 1,8 : 1 umgesetzt wird;
(9) Methyldiallylamin- oder Dimethyldiallylammonium-Cyclopolymeren, beispielsweise den Polymeren, die als Hauptbestandteil der Kette Einheiten der Formeln (III) oder (IV) aufweisen: 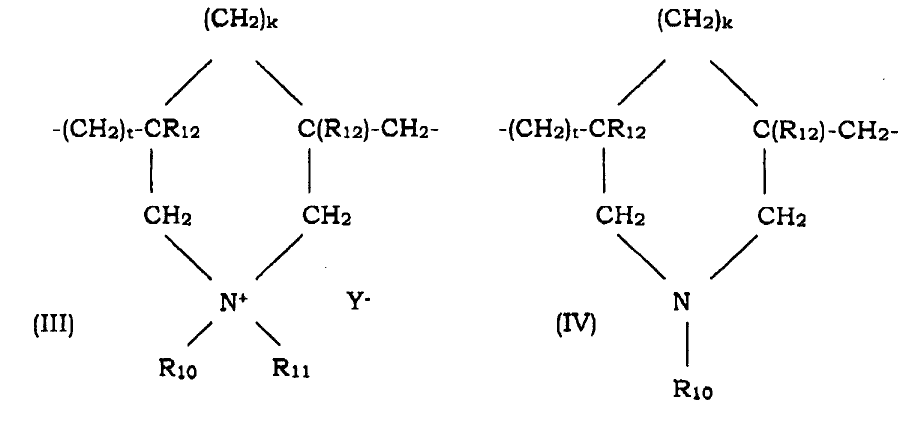 worin k und t 0 oder 1 bedeuten und die Summe k + t l ist; R₁₂ Wasserstoff oder Methyl bedeutet; die Gruppen R₁₀ und R₁₁ unabhängig voneinander eine Alkylgruppe mit 1 bis 22 Kohlenstoffatomen, eine Hydroxyalkylgruppe, worin die Alkylgruppe vorzugsweise 1 bis 5 Kohlenstoffatome aufweist, oder eine niedere Amidoalkylgruppe bedeuten oder R₁₀ und R₁₁ gemeinsam mit dem Stickstoffatom, an das sie gebunden sind, heterocyclische Gruppen, wie Piperidinyl oder Morpholinyl, bilden können; und Y- ein Anion ist, beispielsweise Bromid, Chlorid, Acetat, Borat, Citrat, Tartrat, Bisulfat, Bisulfit, Sulfat oder Phosphat;
(10) Quartären Diammoniumpolymere mit wiederkehrenden Einheiten der folgenden Formel: 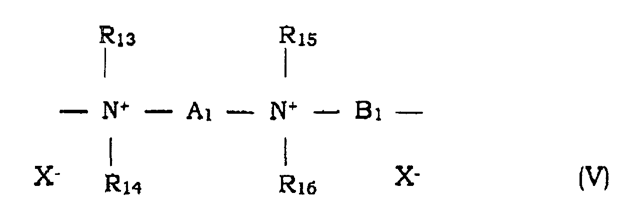 wobei in der Formel (V) die Gruppen R₁₃, R₁₄, R₁₅ und R₁₆, die identisch oder voneinander verschieden sind, aliphatische, alicyclische oder arylaliphatische Gruppen mit 1 bis 20 Kohlenstoffatomen oder niedere hydroxyalkylaliphatische Gruppen bedeuten oder worin die Gruppen R₁₃, R₁₄, R₁₅ und R₁₆ gemeinsam oder unabhängig voneinander mit den Stickstoffatomen, an die sie gebunden sind, Heterocyclen bilden, die gegebenenfalls ein zweites, von Stickstoff verschiedenes Heteroatom enthalten, oder worin die Gruppen R₁₃, R₁₄, R₁₅ und R₁₆ eine geradkettige oder verzweigte C₁₋₆-Alkylgruppe bedeuten, die mit einer Nitril-, Ester-, Acyl- oder Amidgruppe oder -CO-O-R₁₇-D oder -CO-NH-R₁₇-D substituiert ist,
worin R₁₇ eine Alkylengruppe und D eine quartäre Ammoniumgruppe bedeutet;
die Gruppen A₁ und B₁ bedeuten Polymethylengruppen mit 2 bis 20 Kohlenstoffatomen, die geradkettig oder verzweigt, gesättigt oder ungesättigt vorliegen können und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäre Ammoniumgruppen, Ureido, Amid oder Ester, und
X- bedeutet ein Anion, das von einer anorganischen oder organischen Säure abgeleitet ist,
wobei die Gruppen A₁ und R₁₃ und R₁₅ mit den beiden Stickstoffatomen, an die sie gebunden sind, einen Piperazinring bilden können; wenn A₁ eine geradkettige oder verzweigte, gesättigte oder ungesättigte Alkylen- oder Hydroxyalkylengruppe bedeutet, kann die Gruppe B₁ auch eine Gruppe (CH₂)ₙ-CO-D-OC-(CH₂)ₙ- bedeuten, worin D bedeutet:
a) eine Glykolgruppe der Formel -O-Z-O-, worin Z eine geradkettige oder verzweigte Kohlenwasserstoffgruppe oder eine Gruppe der folgenden Formeln bedeutet:
-(CH₂-CH₂-O)ₓ-CH₂-CH₂-
-[CH₂-CH(CH₃)-O]_{y}-CH₂-CH(CH₃)-
worin x und y eine ganze Zahl von 1 bis 4 bedeuten und einen wohldefinierten und einzigen Polymerisationsgrad darstellen oder irgendeine Zahl von 1 bis 4 bedeuten und einen mittleren Polymerisationsgrad darstellen;
b) ein bis-sekundäres Diamin, beispielsweise ein Piperazinderivat;
c) ein bis-primäres Diamin der Formel -NH-Y-NH-, worin Y eine geradkettige oder verzweigte Kohlenwasserstoffgruppe oder die zweiwertige Gruppe -CH₂-CH₂-S-S-CH₂-CH₂- bedeutet;
d) die Ureylengruppe der Formel -NH-CO-NH-;
(11) Quartären Polyammoniumpolymeren, die aus Einheiten der Formel (VI) bestehen: 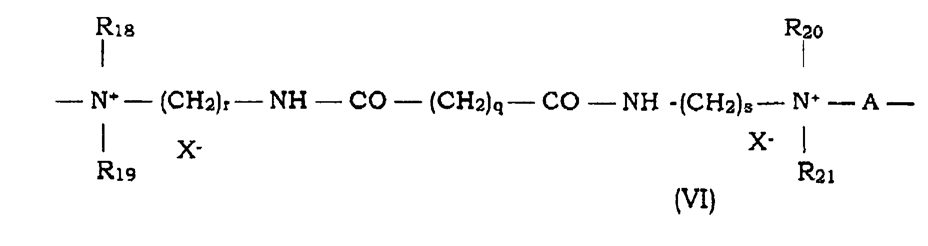 worin bedeuten:
- die Gruppen R₁₈, R₁₉, R₂₀ und R₂₁, die identisch oder voneinander verschieden sind, Wasserstoff oder Methyl, Ethyl, Propyl, β-Hydroxyethyl, β-Hydroxypropyl oder -CH₂CH₂(OCH₂CH₂)ₚOH, worin p 0 oder eine ganze Zahl im Bereich von 1 bis 6 bedeutet, mit der Maßgabe, dass R₁₈, R₁₉, R₂₀ und R₂₁ nicht gleichzeitig ein Wasserstoffatom bedeuten;
- r und s, die identisch oder voneinander verschieden sind, ganze Zahlen im Bereich von 1 bis 6;
- q 0 oder eine ganze Zahl im Bereich von 1 bis 34;
- X ein Halogenatom;
- A eine Dihalogenidgruppe oder vorzugsweise -CH₂-CH₂-O-CH₂-CH₂-;
(12) Homo- oder Copolymeren, die von Acrylsäure oder Methacrylsäure abgeleitet sind und die folgenden Einheiten enthalten: 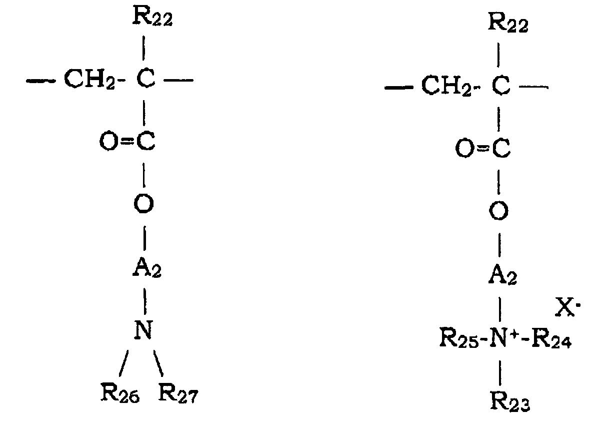 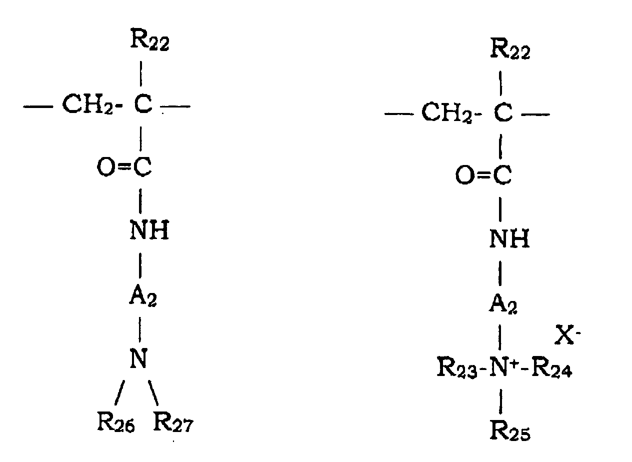 worin bedeuten:
die Gruppen R₂₂ unabhängig H oder CH₃;
die Gruppen A₂ unabhängig eine geradkettige oder verzweigte Alkylgruppe mit 1 bis 6 Kohlenstoffatomen oder eine Hydroxyalkylgruppe mit 1 bis 4 Kohlenstoffatomen;
die Gruppen R₂₃, R₂₄ und R₂₅, die identisch oder voneinander verschieden sind, unabhängig eine Alkylgruppe mit 1 bis 18 Kohlenstoffatomen oder Benzyl;
die Gruppen R₂₆ und R₂₇ Wasserstoff oder eine Alkylgruppe mit 1 bis 6 Kohlenstoffatomen;
X⁻ ein Anion, beispielsweise Methosulfat oder ein Halogenid, wie Chlorid oder Bromid;
(13) Quartären Polymeren von Vinylpyrrolidon und Vinylimidazol;
(14) Polyaminen;
(15) Vernetzten Polymeren von Methacryloyloxyalkyl(C₁₋₄)trialkyl(C₁₋₄)ammoniumsalzen, wie den Polymeren, die durch Homopolymerisation von Dimethylaminoethylmethacrylat, das mit Methylchlorid quaternisiert ist, oder durch Copolymerisation von Acrylamid mit Dimethylaminoethylmethacrylat, das mit Methylchlorid quaternisiert ist, erhalten werden, wobei im Anschluss an die Homopolymerisation oder Copolymerisation mit einer Verbindung mit olefinischer Doppelbindung vernetzt wird, insbesondere mit Methylenbis-acrylamid;
(16) Polyalkyleniminen, insbesondere Polyethyleniminen, Polymeren, die Vinylpyridin- oder Vinylpyridinium-Einheiten enthalten, Kondensaten von Polyaminen und Epichlorhydrin, quartären Polyureylenen und Chitinderivaten.

11. Zusammensetzung nach Anspruch 10, **dadurch gekennzeichnet, dass** das kationische Polymer ausgewählt ist unter:
- Cyclopolymeren, insbesondere Diallyldimethylammoniumsalz-Homopolymeren und Diallyldimethylammoniumsalz/Acrylamid-Copolymeren,
- gegebenenfalls vernetzten Homopolymeren und Copolymeren von (Meth)acryloyloxyethyltrimethylammoniumsalzen,
- Copolymeren von Vinylpyrrolidon und Vinylimidazolsalzen,
- Polymeren, die aus wiederkehrenden Einheiten der folgenden Formel bestehen: 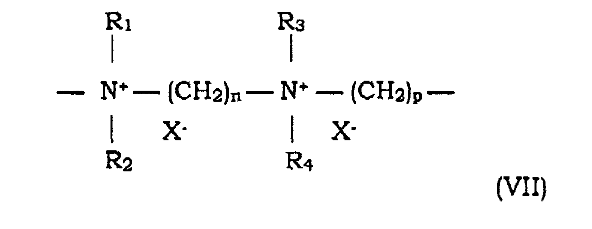 wobei die Gruppen R₁, R₂, R₃ und R₄, die gleich oder verschieden sind, Alkyl- oder Hydroxyalkylgruppen mit etwa 1 bis 4 Kohlenstoffatomen bedeuten, n und p ganze Zahlen sind, die im Bereich von etwa 2 bis 20 liegen, und X⁻ ein von einer anorganischen oder organischen Säure abgeleitetes Anion ist.

12. Zusammensetzung nach einem der Ansprüche 1 bis 11, **dadurch gekennzeichnet, dass** das kationische Polymer 0,05 bis 10 Gew.-%, vorzugsweise 0,1 bis 5 Gew.-% und noch bevorzugter 0,25 bis 3 Gew.-% des Gesamtgewichts der Zusammensetzung ausmacht.

13. Zusammensetzung nach einem der Ansprüche 1 bis 12, **dadurch gekennzeichnet, dass** die wasserlöslichen Salze ein- oder mehrwertige Metallsalze von anorganischen oder organischen Säuren sind.

14. Zusammensetzung nach Anspruch 13, **dadurch gekennzeichnet, dass** die wasserlöslichen Salze unter Natriumchlorid, Kaliumchlorid, Calciumchlorid, Magnesiumsulfat, Natriumcitrat und den Natriumsalzen von Phosphorsäure ausgewählt sind.

15. Zusammensetzung nach einem der Ansprüche 1 bis 14, **dadurch gekennzeichnet, dass** die wasserlöslichen Salze in Konzentrationen von 0,1 bis 10 Gew.-% und vorzugsweise 0,5 bis 5 Gew.-%, bezogen auf das Gesamtgewicht der Zusammensetzung, enthalten sind.

16. Zusammensetzung nach einem der Ansprüche 1 bis 15, **dadurch gekennzeichnet, dass** es sich bei den ein- oder mehrwertigen wasserlöslichen Alkoholen um niedere Alkohole mit 1 bis 6 Kohlenstoffatomen, wie Ethanol, Isopropanol, *t*-Butanol, *n*-Butanol, Polyole, wie Alkylenglykole, beispielsweise Propylenglykol, Glycerin und Polyalkylenglykole, und Glykolether handelt.

17. Zusammensetzung nach einem der Ansprüche 1 bis 16, **dadurch gekennzeichnet, dass** die wasserlöslichen Alkohole in Konzentrationen im Allgemeinen von 0,1 bis 20 Gew.-% und vorzugsweise 0,2 bis 10 Gew.-%, bezogen auf das Gesamtgewicht der Zusammensetzung, enthalten sind.

18. Zusammensetzung nach einem der Ansprüche 1 bis 17, **dadurch gekennzeichnet, dass** sie ferner einen oder mehrere Zusatzstoffe enthält, die unter den kationischen grenzflächenaktiven Stoffen, anionischen, nichtionischen oder amphoteren Polymeren, Proteinen, Proteinhydrolysaten, Ceramiden, Pseudoceramiden, Fettsäuren mit linearen oder verzweigten C₁₆₋₄₀-Fettketten, wie 18-Methyleicosansäure, Hydroxysäuren, Vitaminen, Panthenol, flüchtigen oder nicht flüchtigen Siliconen, die in dem Medium löslich oder unlöslich sind, UV-Filtern, Hydratisierungsmitteln, Wirkstoffen gegen Schuppen oder gegen Seborrhoe, Radikalfängern für freie Radikale und deren Gemischen ausgewählt sind.

19. Verwendung einer Zusammensetzung nach einem der Ansprüche 1 bis 18 zur Reinigung und/oder zum Abschminken und/ oder zum Konditionieren von Keratinsubstanzen.

20. Verfahren zur Reinigung und/oder Konditionierung von Keratinsubstanzen, wie der Haare, das darin besteht, auf die feuchten Keratinsubstanzen eine wirksame Menge einer Zusammensetzung nach einem der Ansprüche 1 bis 18 aufzutragen und anschleißend gegebenenfalls nach einer Einwirkzeit mit Wasser zu spülen.

## Claims

1. Detergent and conditioning cosmetic composition, **characterized in that** it comprises, in a cosmetically acceptable aqueous medium, (A) a washing base comprising at least one anionic surfactant, at least one amphoteric surfactant, (B) at least one oil of polyolefin type with a molecular weight of less than 450, (C) at least one cationic polymer, (D) at least one water-soluble salt and/or one mono- or polyhydroxylated water-soluble alcohol, the anionic surfactant/amphoteric surfactant weight ratio being less than or equal to 3.

2. Composition according to Claim 1, **characterized in that** the said washing base is present in a weight content of between 4% and 50% by weight relative to the total weight of the composition, preferably between 6% and 35% by weight and more preferably between 8% and 25% by weight.

3. Composition according to either of Claims 1 and 2, **characterized in that** the anionic surfactant(s) is(are) present in concentrations ranging from 1 to 30% by weight, preferably from 5 to 20% by weight, relative to the total weight of the composition.

4. Composition according to any one of Claims 1 to 3, **characterized in that** the amphoteric surfactant(s) is(are) present in concentrations ranging from 0.15 to 20% by weight, preferably from 1.5 to 15% by weight, relative to the total weight of the composition.

5. Composition according to any one of Claims 1 to 4, **characterized in that** the anionic surfactant/amphoteric surfactant weight ratio is between 0.2 and 3, more particularly between 0.4 and 2.5.

6. Composition according to any one of Claims 1 to 5, **characterized in that** the said oil of polyolefin type has a molecular weight of between 150 and 350.

7. Composition according to any one of Claims 1 to 6, **characterized in that** the said oil of polyolefin type is a poly-α-olefin.

8. Composition according to Claim 7, **characterized in that** the said oil of poly-α-olefin type is chosen from oils:
- of hydrogenated or non-hydrogenated polybutene type, preferably hydrogenated or non-hydrogenated polyisobutene,
- of hydrogenated or non-hydrogenated polydecene type.

9. Composition according to any one of Claims 1 to 8, **characterized in that** the oil of polyolefin type is present in concentrations of between 0.5 and 15% and preferably between 1 and 10% by weight relative to the total weight of the composition.

10. Composition according to any one of Claims 1 to 9, **characterized in that** the said cationic polymer is chosen from:
(1) Quaternized or non-quaternized vinylpyrrolidone/dialkylaminoalkyl acrylate or methacrylate copolymers.
(2) The cellulose ether derivatives containing quaternary ammonium groups.
(3) Cationic cellulose derivatives such as cellulose copolymers or cellulose derivatives grafted with a water-soluble monomer of quaternary ammonium.
(4) Polysaccharides such as guar gums containing cationic trialkylammonium groups.
(5) Polymers consisting of piperazinyl units and of divalent alkylene or hydroxyalkylene radicals containing straight or branched chains, optionally interrupted by oxygen, sulphur or nitrogen atoms or by aromatic or heterocyclic rings, as well as the oxidation and/or quaternization products of these polymers.
(6) Water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they contain one or more tertiary amine functions, they can be quaternized.
(7) Polyamino amide derivatives resulting from the condensation of polyalkylene polyamines with polycarboxylic acids followed by alkylation with difunctional agents.
(8) Polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms. The molar ratio between the polyalkylene polyamine and the dicarboxylic acid being between 0.8:1 and 1.4:1; the polyamino amide resulting therefrom being reacted with epichlorohydrin in a molar ratio of epichlorohydrin relative to the secondary amine group of the polyamino amide of between 0.5:1 and 1.8:1.
(9) Cyclopolymers of methyldiallylamine or of dimethyldiallylammonium, such as the homopolymers or copolymers containing, as main constituent of the chain, units corresponding to formulae (III) or (IV) : 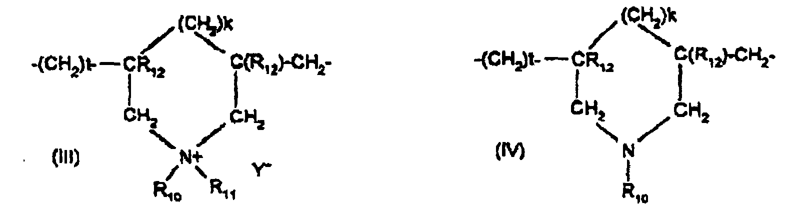 in which formulae k and t are equal to 0 or 1, the sum k + t being equal to 1; R₁₂ denotes a hydrogen atom or a methyl radical; R₁₀ and R₁₁, independently of each other, denote an alkyl group having from 1 to 22 carbon atoms, a hydroxyalkyl group in which the alkyl group preferably has 1 to 5 carbon atoms, or a lower amidoalkyl group, or R₁₀ and R₁₁ can denote, together with the nitrogen atom to which they are attached, heterocyclic groups such as piperidyl or morpholinyl; Y⁻ is an anion such as bromide, chloride, acetate, borate, citrate, tartrate, bisulphate, bisulphite, sulphate or phosphate.
(10) The quaternary diammonium polymer containing repeating units corresponding to the formula: 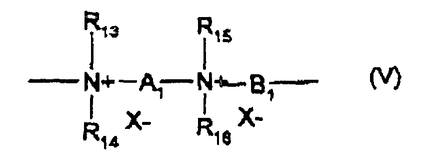 in which formula (V):
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second hetero atom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms which may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
X⁻ denotes an anion derived from an inorganic or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH2)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
-(CH₂-CH₂-O)ₓ-CH₂-CH₂-
-[CH₂-CH(CH₃)-O]_{y}-CH₂-CH(CH₃)-
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon radical, or alternatively the divalent radical
-CH₂-CH₂-S-S-CH₂-CH₂-;
d) a ureylene group of formula: -NH-CO-NH-.
(11) Quaternary polyammonium polymers consisting of units of formula (VI) : 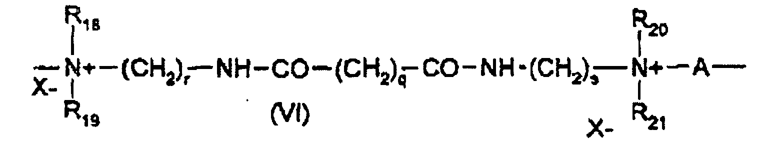 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X denotes a halogen atom,
A denotes a dihalide radical or preferably represents -CH₂-CH₂-O-CH₂-CH₂-.
(12) Homopolymers or copolymers derived from acrylic or methacrylic acids and containing units chosen from: 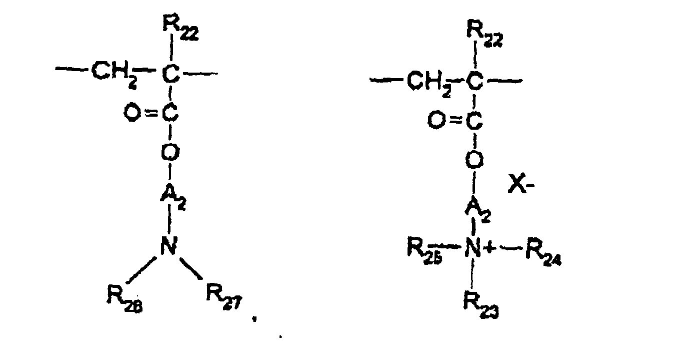 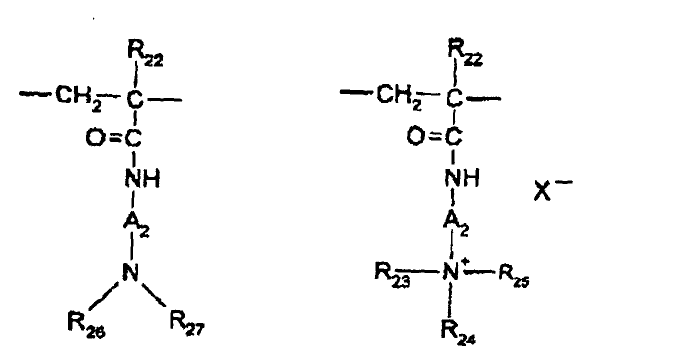 in which the groups R₂₂ independently denote H or CH₃,
the groups A₂ independently denote a linear or branched alkyl group of 1 to 6 carbon atoms or a hydroxyalkyl group of 1 to 4 carbon atoms,
the groups R₂₃, R₂₄ and R₂₅, which may be identical or different, independently denote an alkyl group of 1 to 18 carbon atoms or a benzyl radical,
the groups R₂₆ and R₂₇ represent a hydrogen atom or an alkyl group of 1 to 6 carbon atoms,
X⁻ denotes an anion, for example methosulphate or halide, such as chloride or bromide.
(13) Quaternary polymers of vinylpyrrolidone and of vinylimidazole,
(14) Polyamines,
(15) Crosslinked polymers of methacryloyloxy (C₁-C₄)alkyltri(C₁-C₄)alkylammonium salts such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with a compound containing olefinic unsaturation, in particular methylenebisacrylamide.
(16) Polyalkyleneimines, in particular polyethyleneimines, polymers containing vinylpyridine or vinylpyridinium units, condensates of polyamines and of epichlorohydrin, quaternary polyureylenes and chitin derivatives.

11. Composition according to Claim 10, **characterized in that** the said cationic polymer is chosen from:
- cyclopolymers, in particular homopolymers of a diallyldimethylammonium salt and copolymers of a diallyldimethylammonium salt and of acrylamide,
- optionally crosslinked homopolymers and copolymers of a (meth)acryloyloxyethyltrimethylammonium salt,
- copolymers of vinylpyrrolidone and of a vinylimidazole salt,
- polymers which consist of repeating units corresponding to the formula: 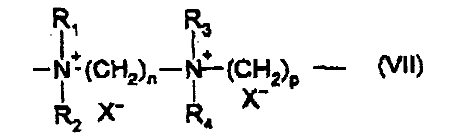
in which R₁, R₂, R₃ and R₄, which may be identical or different, denote an alkyl or hydroxyalkyl radical containing from 1 to 4 carbon atoms approximately, n and p are integers ranging from 2 to 20 approximately and X⁻ is an anion derived from an inorganic or organic acid.

12. Composition according to any one of Claims 1 to 11, **characterized in that** the said cationic polymer represents from 0.05% to 10% by weight, preferably from 0.1% to 5% by weight and even more preferably from 0.25% to 3% by weight, relative to the total weight of the composition.

13. Composition according to any one of Claims 1 to 12, **characterized in that** the water-soluble salts are mono- or divalent metal salts of an inorganic or organic acid.

14. Composition according to Claim 13, **characterized in that** the water-soluble salts are chosen from sodium chloride, potassium chloride, calcium chloride, magnesium sulphate, sodium citrate and the sodium salts of phosphoric acid.

15. Composition according to any one of Claims 1 to 14, **characterized in that** the water-soluble salts are present in concentrations of between 0.1 and 10% by weight and preferably between 0.5 and 5% by weight relative to the total weight of the composition.

16. Composition according to any one of Claims 1 to 15, **characterized in that** the mono- or polyhydroxylated water-soluble alcohols are C₁-C₆ lower alcohols, such as ethanol, isopropanol, tert-butanol, n-butanol, polyols such as alkylene glycols, for instance propylene glycol, glycerol and polyalkylene glycols; glycol ethers.

17. Composition according to any one of Claims 1 to 16, **characterized in that** the water-soluble alcohols are used in concentrations generally of between 0.1 and 20% by weight and more particularly between 0.2 and 10% by weight relative to the total weight of the composition.

18. Composition according to any one of Claims 1 to 17, **characterized in that** it also contains one or more adjuvants chosen from cationic surfactants, anionic or nonionic or amphoteric polymers, proteins, protein hydrolysates, ceramides, pseudoceramides, fatty acids containing linear or branched C₁₆-C₄₀ chains, such as 18-methyleicosanoic acid, hydroxy acids, vitamins, panthenol, volatile or non-volatile silicones, which may be soluble or insoluble in the medium, UV screening agents, moisturizers, antidandruff or anti-seborrhoeic agents, free-radical scavengers and mixtures thereof.

19. Use of a composition as defined in any one of Claims 1 to 18, for cleansing and/or removing make-up from and/or conditioning keratin substances.

20. Process for washing and conditioning keratin substances such as the hair, which consists in applying an effective amount of a composition as defined in any one of Claims 1 to 18 to the said wet substances and then in rinsing with water, after an optional waiting time.
